# EUROPEAN PATENT APPLICATION

(11) **EP 1 384 718 A1**
(43) Date of publication of application: **28.01.2004**
(21) Application number: 03102223.9
(22) Date of filing: 18.07.2003
(51) Int. Cl.: C07D 291/02, C07D 291/08

(54) **Intramolecular amidation of sulfamate esters catalyzed by metalloporphyrins**

(30) Priority: 23.07.2002 US 202581
(71) Applicant: The University of Hong Kong, Hong Kong (CN)
(72) Inventor: Che, Chiming, Hong Kong (CN); Liang, Jianglin, Hong Kong (CN)
(74) Representative: Epping Hermann & Fischer

(57) **Abstract**

The present invention relates to novel intramolecular amidation processes for substrates such as sulfamate esters using chiral and non-chiral metalloporphyrin complexes, which can maximize catalytic activity and enhance efficiency, stereoselectivity and speed of amidation of these substrates. The intramolecular amidation of sulfamate ester exhibits excellent cis-selectivity, affording cyclic sulfamidates with high ee values catalyzed by chiral metalloporphyrin.

## Description

### FIELD OF THE INVENTION

The invention relates to methods for the direct intramolecular amidation of sulfamate esters affording cyclic sulfamidates. More particularly, the method represents an example of asymmetric intramolecular amidation of sulfamate ester with high ee values.

### BACKGROUND OF THE INVENTION

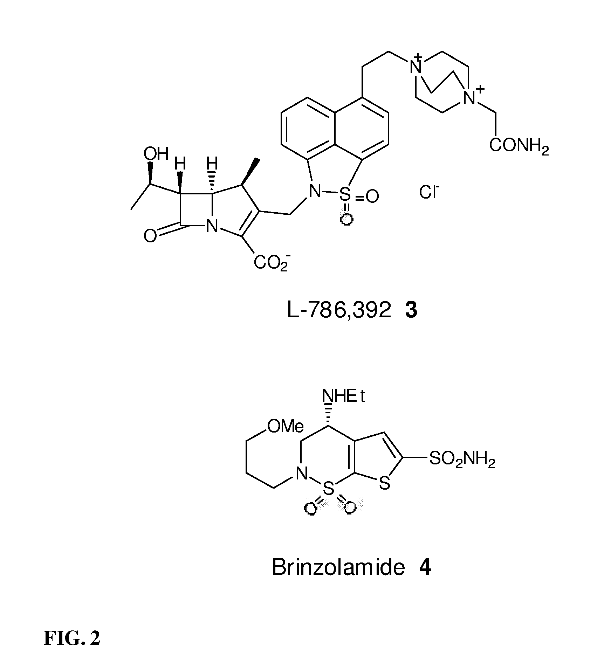

Cyclic sulfamidate or sulfonamide is useful building block for drug discovery. Major recent pharmaceutical applications include the carbapenem antibiotic L-786,392 (Compound **3**, see FIG. 2), and brinzolamide (Compound **4**, see FIG. 2) for the treatment of glaucoma (Rosen *et al., Science* (1999), 283, 703; Dauban *et al., Org. Lett.* (2000), 2, 2327; Dauban *et al.,* and *Tetrahedron Lett.* (2001), 42, 1037. FIG. 2). Cyclic sulfamidate has also been utilized in the preparation of amino acids (Baldwin *et al., Tetrahedron: Asymmetry* (1990), 1, 881; Boulton *et al., J. Chem. Soc., Perkin Trans.* (1999), 1, 1421; and Halcomb *et al., J. Am. Chem. Soc.* (2002), 124, 2534), and serves as chiral auxiliaries (Oppolzer *et al., Tetrahedron Lett.* (1994), 35, 3509; Ahn *et al., Tetrahedron Lett.* (1998), 39, 6321; and Lin *et al., Tetrahedron* (1999), 55, 13983).

Catalytic intramolecular amidation was pioneered by Breslow in 1983 using transition-metal porphyrin complexes and rhodium acetate as catalysts yielding cyclic sulfonamides (Breslow *et al., J. Am. Chem. Soc.* (1983), 105, 6728).
Recent research by Du Bois showed that rhodium acetate served as an efficient catalyst for intramolecular amidation of sulfamate esters affording corresponding cyclic sulfamidates in good to high yield (Du Bois *et al., J. Am. Chem. Soc.* (2001), 123, 6935). However, challenge remains to seek more stereoselective catalysts for the synthesis of optically active cyclic sulfamidates. There has been no report on asymmetric intramolecular amidation of such substrates using chiral catalysts.

On the other hand, metalloporphyrin-catalyzed intermolecular nitrogen-atom transfer has attracted considerable attention not only because of its unique relationship to heme-containing enzymes but also because of its unusually high selectivity and catalytic turnover (Che *et al., Org. Lett.* (2000), 2, 2233). Moderate ee values have been obtained for asymmetric aziridination of alkenes and amidation of saturated C-H bonds (Che *et al., Chem. Comm.* (1997), 2373; Che *et al., Chem. Comm.* (1999), 2377; Marchon *et al., Chem. Comm.* (1999), 989; and Che *et al., Chem. Eur.* J. (2002), 1563). The successful isolation of bis(tosylimido) ruthenium porphyrin provides useful insight into the mechanism of ruthenium porphyrin-catalyzed intramolecular nitrogen-atom transfer reactions (Che *et al., J. Am. Chem. Soc.* (1999), 121, 9120; and Che *et al., Chem. Eur. J.* (2002), 1563).

The present invention provides for the first time (1) intramolecular amidation of sulfamate esters catalyzed by a metalloporphyrin and (2) asymmetric intramolecular amidation of sulfamidate esters catalyzed by a transition-metal complex. The target cyclic sulfamidates can be easily converted to α or β amino alcohol (Du Bois *et al., J. Am. Chem. Soc.* (2001), 123, 6935), which is an important precursor for drug synthesis and for the synthesis chiral ligands for asymmetric catalysis (Kajiro *et al., Synlett* (1998), 51; Davies *et al., Tetrahedron Lett.* (1996), 37, 813; Ghosh *et al., J. Am. Chem. Soc.* (1996), 118, 2527).

### SUMMARY OF THE INVENTION

The present invention relates to a method for synthesizing a cyclic sulfamidate from a sulfamate ester comprising the step of catalyzing the reaction of an oxidant and a base with said sulfamate ester with a catalytic amount of metalloporphyrin as catalyst for producing the cyclic sulfamidate.

In a preferred embodiment, the sulfamate ester is a compound comprising a sulfonylamide functional group.

In a preferred embodiment, the oxidant is selected from the group consisting of PhI(OAc)₂, PhIO, and NBS.

In a preferred embodiment, the solvent for the reaction is acetonitrile, DMF, CH₂Cl₂ or benzene.

In a preferred embodiment, the base is Al₂O₃, MgO, ZnO, K₂CO₃, and KOH.

In a preferred embodiment, the catalyst has the following structure: 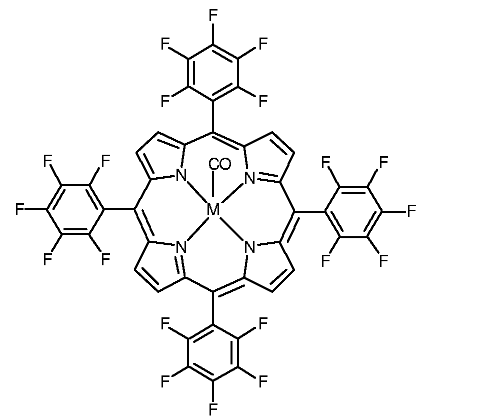 wherein M is a metal, preferably a transition metal, most preferably ruthenium.

In another preferred embodiment, the catalyst has the following structure: 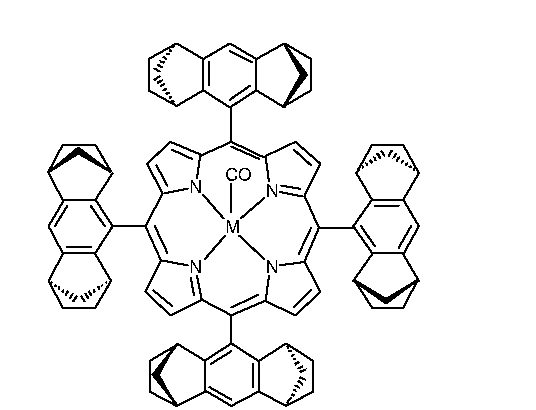 wherein M is a metal, preferably a transition metal, most preferably ruthenium.

### BRIEF DESCRIPTION OF THE FIGURES

**FIG. 1** provides representative examples of the metalloporphyrin catalysts capable of catalyzing intramolecular amidation of sulfamate esters with high efficiency and high diastereo- or enantio- selectivity.
**FIG. 2** provides representative examples of drugs containing cyclic sulfonamide unit.

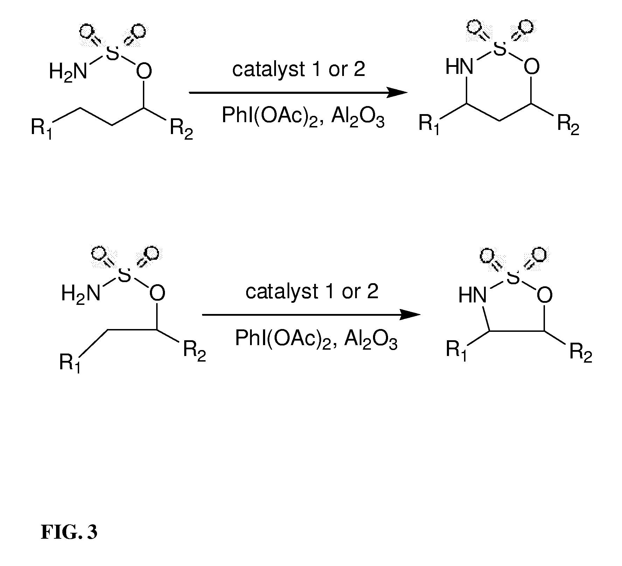

**FIG. 3** illustrates the described method involving the direct intramolecular amidation of sulfamate esters using metalloporphyrin as a general and efficient catalyst.
**FIG. 4** provides representative examples of intramolecular amidation of sulfamate esters catalyzed by electron-deficient ruthenium porphyrin yielding the corresponding cyclic sulfamidates in good to excellent yields with excellent diastereoselectivity.
**FIG. 5** provides representative examples of intramolecular amidation of sulfamate esters catalyzed by electron-deficient ruthenium porphyrin with high turnover numbers.
**FIG. 6** provides representative examples of asymmetric intramolecular amidation of sulfamate esters catalyzed by chiral ruthenium porphyrin with high enantioselectivity.
**FIG 7.** provides representative examples of pharmaceutical application of α-amino alcohol.

### DETAILED DESCRIPTION OF THE INVENTION

The present invention provides a non-chiral metalloporphyrin catalyst represented by the structure: 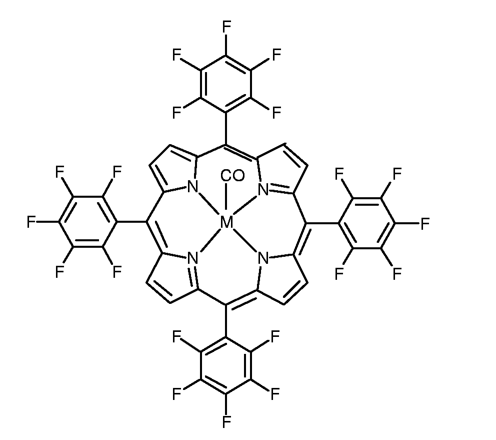 and a chiral metalloporphyrin catalyst represented by the structure: 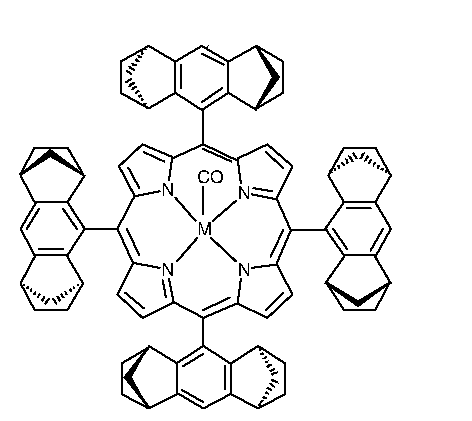 wherein M is a metal, preferably a transition metal such as ruthenium, manganese, iron, osmium, copper or cobalt, and most preferably ruthenium.

In one embodiment of this invention, the metalloporphyrin is a transition metal metalloporphyrin, such as ruthenium, manganese, iron, osmium, copper or cobalt metalloporphyrin.

In one embodiment of this invention, the porphyrin ligand is a tetraphenylporphyrin and the phenyl rings are attached at the meso positions of the porphyrin.

In one embodiment of the present invention, catalysts are capable of exhibiting both regioselectivity and stereoselectivity.

In one embodiment of the present invention, the catalyst is capable of selectively catalyzing intramolecular amidation of saturated C-H bonds. In another embodiment of the present invention, the catalyst is capable of catalyzing asymmetric intramolecular amidation of saturated C-H bonds. In one embodiment of this invention, the stereoselectivity is for the formation of only cis-configuration cyclic sulfamidates.

Additionally, the present invention provides a method for preparation of cyclic sulfamidates with the catalysts from sulfamate esters. Further, the present invention provides a method for producing a cis-cyclic sulfamidate with the catalyst. The present invention also provides a method for producing an optically active cyclic sulfamidate with the catalyst.

In an embodiment of this invention, the substrate is a sulfamate ester, a sulfamate ester derivative, or a hydrocarbon containing sulfonylamide group.

A number of solvents known in the art can used to carry out the reaction. Such solvents include but are not limited to acetonitrile, DMF, CH₂Cl₂, and benzene.

A number of bases know in the art can be used. Such bases include but are not limted to Al₂O₃, MgO, ZnO, K₂CO₃, and KOH.

As used herein, the term "stereoselective" refers to selection of an optical isomer and the term "enantioselectivity" represents the maximal asymmetric induction and minimal racemization of the optically active products. The term "turnover" refers to the relative number of molecules of products per number of molecules of catalyst prior to the exhaustion of a given reaction.

Various publications are cited herein, the disclosures of which are incorporated by reference in their entirety for all purposes.

Having described the invention, the following examples are included to illustrate the benefits of the present invention. The examples are only illustrative and are not meant to unduly limit the scope of the present invention.

### EXAMPLES

### EXAMPLE 1

### Intramolecular Amidation of Sulfamate Esters Catalyzed by Electron-Deficient Ruthenium Porphyrin (Compound/Catalyst 1)

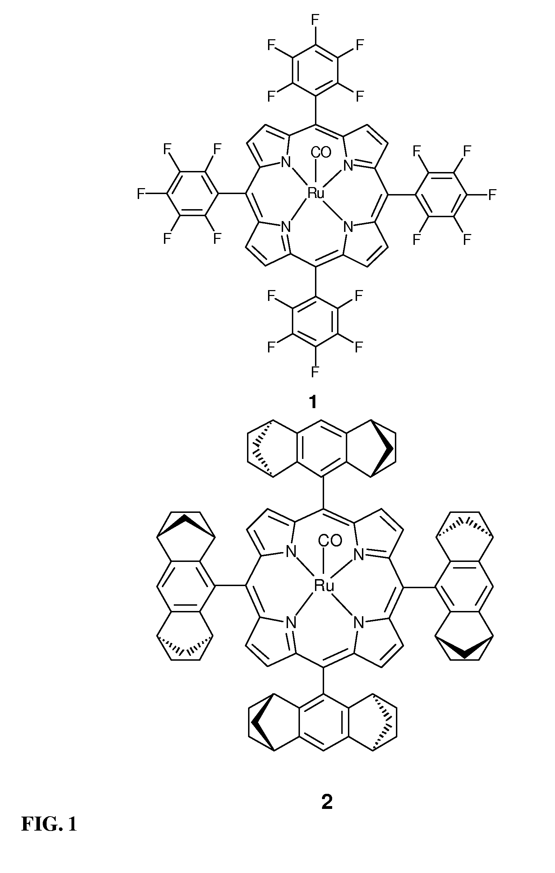

The invention relates to a direct method of synthesis of cyclic sulfamidates using ruthenium porphyrin (Compound/Catalyst **1,** see FIG. 1, prepared according to: Murahashi *et al., Tetrahedron Lett.* 1995, 36,8059; Groves *et al., J. Am. Chem. Soc.* 1996, 118, 8961) as a general and effective catalyst for the direct intramolecular amidation of sulfamate esters.

Typical conditions employ 1.5% of Catalyst **1**, 1 equiv. of sulfamate ester, 2 equiv. of Phi(OAc)₂, 2.5 equiv. of anhydrous Al₂O₃ (pH = 7-7.4) in distilled dichloromethane under argon at 40 °C for 2 hours. The commercially available Al₂O₃ was dried to constant weight at 250 °C for 12 hours. Dichloromethane was freshly distilled from CaH₂ immediately prior to use. The reaction mixture was cooled to 25° C, diluted with 5 mL of CH₂Cl₂, and filtered through a pad of celite. The filter cake was rinsed with 2 × 5 mL of CH₂Cl₂ and the combined filtrates were evaporated under reduced pressure. The residue was purified by chromatography on silica gel (230-400 mesh) to afford the corresponding cyclic sulfamidates. AcOH generated as a by-product from PhI(OAc)₂ should be scavenged from the reaction mixture by addition of base. Following a series of control experiments, Al₂O₃ was proved the best among MgO, ZnO, K₂CO₃, Al₂O₃ and KOH, giving the highest yields.

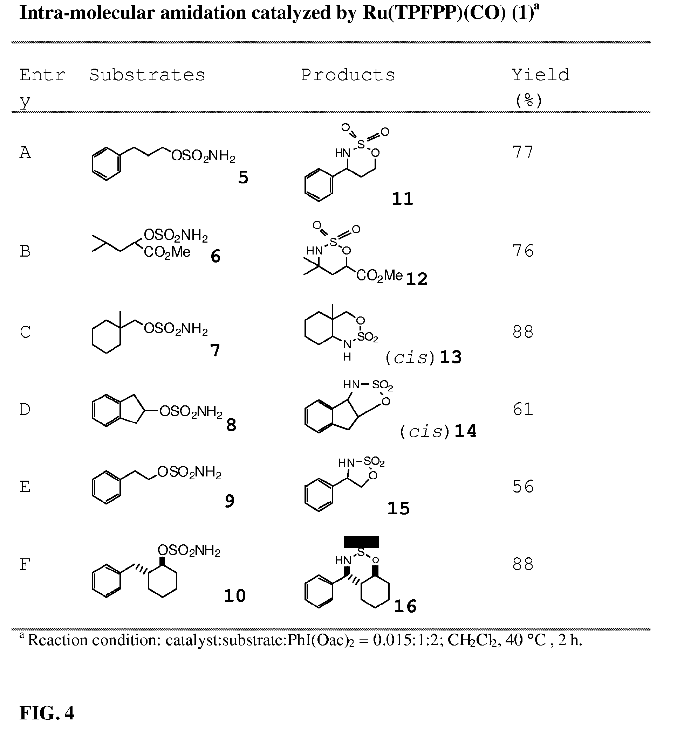

With only 1.5% catalyst loading, sulfamate esters (Compounds/Substrates **5-10,** see FIG. 4) were converted into corresponding cyclic sulfamidates (Compounds/Products **11-16,** see FIG. 4) with good to high yields (see FIG. 4). The highest yield (88%) was achieved on the intramolecular amidation of Substrates **7** and **10**. Catalyst **1** not only shows high catalytic efficiency, but also shows excellent cis-selectivity. For Substrates **7, 8** and **10**, only cis-cyclic sulfamidates **12, 13** and **16** were obtained respectively, and trans-cyclic sulfamidates were undetectable. This shows that ruthenium porphyrin **1** has better stereoselectivity than rhodium acetate (cis and trans mixture were obtained when Substrate **8** was catalyzed by rhodium acetate. The ratio of cis/trans is 8:1. See: Du Bois *et al., J. Am. Chem. Soc.* 2001, 123, 6935). The oxidant used in the catalytic reaction is PhI(OAc)₂, which is commercially available. For Substrates **5-7,** and **10**, six- rather than five-membered heterocycles **10-12** and **16** were formed in high yields. For substrates **8** and **9,** there is no possibility to form six-membered ring from direct intramolecular amidation of their saturated C-H bonds, so five-membered ring products **14** and **15** were obtained in lower yields compared with **11**-**13** and **16**.

All the target cyclic sulfamidates were characterized by ¹H, ¹³C and NOESY NMR and HRMS. The spectral data of Products **11-14** are identical with those reported in the literature (see: Du Bois *et al., J. Am. Chem. Soc.* 2001, 123, 6935). Spectral data for Compound **9** are: ¹H NMR (CDCl₃, 400 MHz) : δ = 7.29 (m, 5H), 4.82 (s, 2H), 4.37 (t, *J* = 9.3 Hz, 2H), 3.03 (t, *J* = 9.2 Hz, 2H) ; ¹³C NMR (CDCl₃, 100 MHz) : δ = 136.4, 128.9, 128.7, 127.0, 71.4, 35.2. HRMS (EI) calcd. for C₈H₁₁NO₃S: 201.0460, found: 201.0456. Spectral data for Compound **10** are: ¹H NMR (CDCl₃, 400 MHz) 7.23 (m, 5H), 4.66 (s, 2H), 4.34 (m, 1H), 3.15 (dd, 1H, *J* = 13.6 Hz), 2.32 (m, 2H), 1.10-1.86 (m, 8H) ppm; ¹³C NMR (CDCl₃, 100 MHz) 140.0, 129.2, 128.3, 126.0, 87.2, 44.0, 38.9, 32.4, 30.0, 24.5, 24.4 ppm; HRMS (EI) calcd. for C₁₃H₁₉NO₃S: 269.1087, found: 269.1090. Spectral data for Compound **15** are: ¹H NMR (CDCl₃, 400 MHz) 7.43 (m, 5H), 5.07 (m, 1H), 4.84 (m, 2H), 4.45 (t, *J* = 6.5 Hz, 1H) ppm; ¹³C NMR (CDCl₃, 100 MHz) 135.3, 129.5, 129.4, 126.7, 75.0, 59.6 ppm; HRMS (EI) calcd. for C₈H₉NSO₃: 199.0303, found: 199.0297. Spectral data for Compound **16** are: ¹H NMR (CDCl₃, 400 MHz) 7.37 (m, 5H), 4.60 (m, 1H), 4.39 (m, 2H), 2.14 (m, 1H), 1.10-1.85 (m, 8H) ppm; ¹³C NMR (CDCl₃, 100 MHz) 136.7, 129.3, 129.1, 127.3, 86.9, 64.3, 45.3, 31.8, 29.7, 24.8, 24.4 ppm; HRMS (EI) calcd. for C₁₃H₁₇NO₃S: 267.0929, found: 267.0935.

### EXAMPLE 2

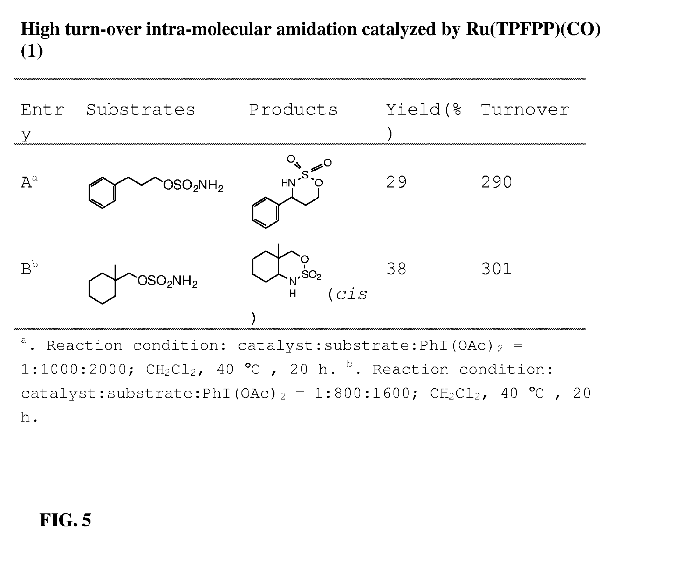

Turnover refers to the relative number of molecules of products per number of molecules of catalyst prior to the exhaustion of a given reaction and shows a very important aspect of catalyst efficiency. The turnover numbers of rhodium catalyst don't excess 50 (see: Du Bois *et al., J. Am. Chem. Soc.* (2001), 123, 6935). With electron-deficient ruthenium porphyrin **1** as catalyst, intramolecular amidation of **5** and **7** afforded 290 and 301 turnover numbers, respectively (FIG. 5). This shows that catalyst **1** is more robust than rhodium catalysts (the reaction condition are almost the same as those example 1, except that lower catalyst loading was employed in example 2. See the footnote of FIG. 5).

### EXAMPLE 3

### Asymmetric Intramolecular Amidation of Sulfamate Ester Catalyzed by

### Chiral Ruthenium Porphyrin (Compound/Catalyst 2)

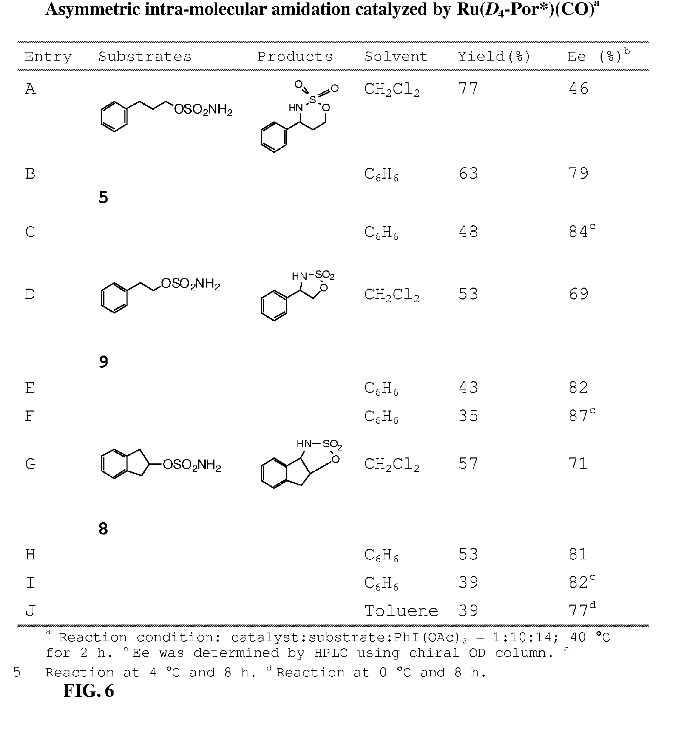

With chiral ruthenium porphyrin (Compound/Catalyst **2** in FIG. 1) (prepared according to: Che *et al., Chem. Comm.* (1997), 1205) as catalyst, sulfamate esters (Compounds/Substrates **5, 8** and **9** in FIG. 4) undergo enantioselective C-H insertion yielding corresponding cyclic sulfamidates with high ee values (FIG. 6). In order to reduce the amount of by-product, the ratio between substrate and PhI(OAc)₂ was decreased from 2 to 1.4. Solvent has a very important effect on the ee values. For example, sulfamate ester **5** was converted into **11** with 46% ee when the reaction was performed in CH₂Cl₂ (entry A of FIG. 6). But the ee value increased sharply to 79% when benzene was used as solvent (entry B of FIG. 6). Same conclusion can be obtained for Substrates **8** and **9**.

The reaction temperature has effect on the ee values as well. When benzene was used as solvent, lowering reaction temperature resulted in increase in ee values (entries B and C in FIG. 6: from 79 to 84%; entries E and F in FIG. 6: from 82 to 87%; entries H and I in FIG. 6: from 81 to 82%).

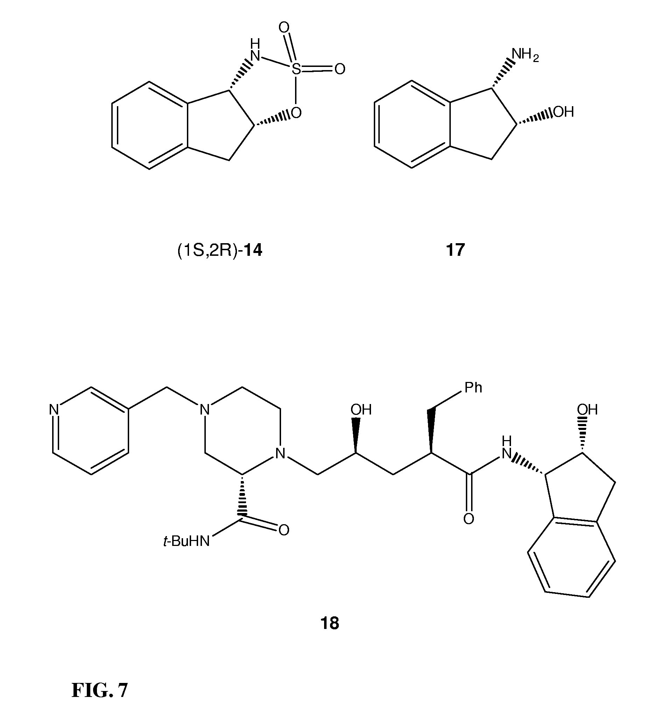

The present invention provides a powerful method for the synthesis of chiral cyclic sulfamidates. These compounds are potentially useful for the synthesis of optically active α or β-amino alcohol, which is of particular importance for drug synthesis. For example, optically-active Compound **17** (see FIG. 7) is currently receiving considerable attention as the key component of HIV protease inhibitor, indinavir Compound **18** (see: Hiyama *et al., Synlett* (1998), 51. FIG. 7). Compound **17** can be easily prepared from (1S,2R)-Compound **14** (see FIG. 7) after hydrolysis (see: Du Bois *et al., J. Am. Chem. Soc.* (2001), 123, 6935). From commercially available and non-chiral 2-indanol, Compound **17** can be obtained through 3 steps. However, it took eight steps to yield Compound **17** from chiral amino acid as starting material (see: Hiyama *et al., Synlett* (1998), 51).

## Claims

1. A method for synthesizing a cyclic sulfamidate from a sulfamate ester comprising the step of catalyzing the reaction of an oxidant and a base with said sulfamate ester with a catalytic amount of metalloporphyrin as catalyst for producing the cyclic sulfamidate.

2. The method according to claim 1 wherein the sulfamate ester is a compound comprising a sulfonylamide functional group.

3. The method according to claim 1 wherein the oxidant is selected from the group consisting of PhI(OAc)₂, PhIO, and NBS.

4. The method according to claim 1 further comprising an organic solvent selected from the group consisting of acetonitrile, DMF, CH₂Cl₂, and benzene.

5. The method according to claim 1 wherein the base is selected from the group consisting of Al₂O₃, MgO, ZnO, K₂CO₃, and KOH.

6. The method accoring to claim 1 wherein the metalloporphyrin is a transition metal metalloporphyrin.

7. The method according to claim 6 wherein the transition metal metalloporphyrin is selected from the group consisting of ruthenium, manganese, iron, cobalt, copper and osmium metalloporphyrin.

8. The method according to claim 7, wherein the metalloporphyrin is ruthenium porphyrin.

9. The method according to claim 1 wherein the metalloporphyrin catalyst has the structure: 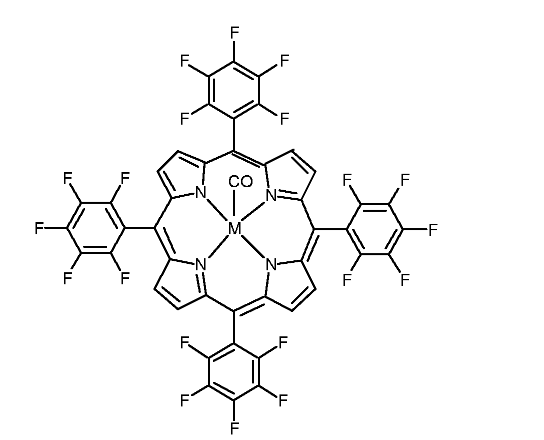 wherein M represents a metal.

10. The method of claim 9 wherein M is ruthenium.

11. The method of claim 9 wherein the catalyst exhibits cis-selectivity.

12. The method according to claim 1 wherein the metalloporphyrin catalyst has the structure: 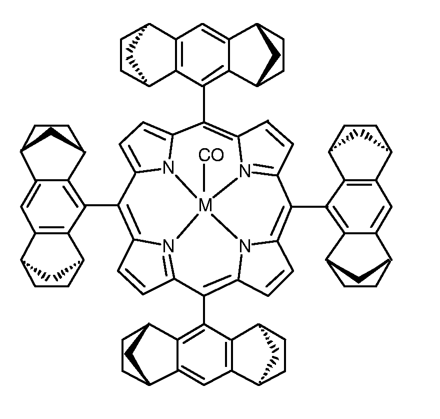 wherein M represents a metal.

13. The method of claim 12 wherein M is ruthenium.

14. The method of claim 12 wherein the catalyst exhibit cis-selectivity.

15. The method of claim 12 wherein the catalyst exhibits enantioselctivity and yields corresponding cyclic sulfamidate with high ee value.
